# EUROPEAN PATENT SPECIFICATION

(11) **EP 2 517 017 B1**
(45) Date of publication and mention of the grant of the patent: **21.01.2015**
(21) Application number: 10798829.7
(22) Date of filing: 20.12.2010
(51) Int. Cl.: G01N 33/68

(54) **BIOMARKERS**
BIOMARKER
BIOMARQUEURS

(30) Priority: 21.12.2009 GB 0922240
(43) Date of publication of application: 31.10.2012
(73) Proprietor: Cambridge Enterprise Ltd., Cambridge Cambridgeshire CB2 1TN (GB)
(72) Inventor: BAHN, Sabine, Cambridge Cambridgeshire CB2 1QT (GB); SCHWARZ, Emanuel, Cambridge Cambridgeshire CB2 1QT (GB); LEVIN, Yishai, Hadera 38523 (IL)
(74) Representative: Gibson, Mark
(86) International application number: PCT/GB2010/052159
(87) International publication number: WO 2011/077129

(56) References cited:
- WO-A1-2009/077763

## Description

### FIELD OF THE INVENTION

The invention relates to a method of diagnosing or monitoring schizophrenia or other psychotic disorder.

### BACKGROUND OF THE INVENTION

Schizophrenia is a psychiatric diagnosis that describes a mental disorder characterized by abnormalities in the perception or expression of reality. It most commonly manifests as auditory hallucinations, paranoid or bizarre delusions, or disorganized speech and thinking with significant social or occupational dysfunction. Onset of symptoms typically occurs in young adulthood, with approximately 0.4-0.6% of the population affected. Diagnosis is based on the patient's self-reported experiences and observed behavior. No laboratory test for schizophrenia currently exists.

Studies suggest that genetics, early environment, neurobiology, psychological and social processes are important contributory factors; some recreational and prescription drugs appear to cause or worsen symptoms. Current psychiatric research is focused on the role of neurobiology, but no single organic cause has been found. Due to the many possible combinations of symptoms, there is debate about whether the diagnosis represents a single disorder or a number of discrete syndromes.

The disorder is thought to mainly affect cognition, but it also usually contributes to chronic problems with behavior and emotion. People with schizophrenia are likely to have additional (comorbid) conditions, including major depression and anxiety disorders; the lifetime occurrence of substance abuse is around 40%. Social problems, such as long-term unemployment, poverty and homelessness, are common. Furthermore, the average life expectancy of people with the disorder is 10 to 12 years less than those without, due to increased physical health problems and a higher suicide rate.

An important utility of biomarkers for psychotic disorders is their response to medication. Administration of antipsychotics remains a subjective process, relying solely on the experience of clinicians. Furthermore, the development of antipsychotic drugs has been based on chance findings often with little relation to the background driving the observations.

Schizophrenia is treated primarily with antipsychotic medications which are also referred to as neuroleptic drugs or neuroleptics. Newer antipsychotic agents such as Clozapine, Olanzapine, Quetiapine or Risperidone are thought to be more effective in improving negative symptoms of psychotic disorders than older medication like Chlorpromazine. Furthermore, they induce less extrapyramidal side effects (EPS) which are movement disorders resulting from antipsychotic treatment.

The history of neuroleptics dates back to the late 19th century. The flourishing dye industry catalyzed development of new chemicals that lay the background to modern day atypical antipsychotics. Developments in anti malaria, antihistamine and anaesthetic compounds also produced various neuroleptics. The common phenomenon to all these processes is a fundamental lack of understanding of the biological mechanisms and pathways that these drugs affect, apart from the observation that they prominently block D2 receptors in the striatum.

There is therefore a pressing need for objective molecular readouts that can diagnose schizophrenia or other psychotic disorders and furthermore indicate whether a patient is responding to medication, as well as for predicting prognosis.

### SUMMARY OF THE INVENTION

According to a first aspect of the invention, there is provided the use of Alpha 1 Antitrypsin, Apolipoprotein A1, Cortisol, Ferritin and IgA as a specific panel of biomarkers for schizophrenia or other psychotic disorder, or predisposition thereto.

According to a further aspect of the invention, there is provided a method of diagnosing or monitoring schizophrenia or predisposition thereto, comprising detecting and/or quantifying, in a sample from a test subject, the panel of biomarkers defined herein.

According to a further aspect of the invention, there is provided a method of monitoring efficacy of a therapy in a subject having, suspected of having, or of being predisposed to schizophrenia, comprising detecting and/or quantifying, in a sample from said subject, the panel of biomarkers defined herein.

Provided are ligands, such as naturally occurring or chemically synthesised compounds, capable of specific binding to the analyte biomarker. A ligand may comprise a peptide, an antibody or a fragment thereof, or an aptamer or oligonucleotide, capable of specific binding to the analyte biomarker. The antibody can be a monoclonal antibody or a fragment thereof capable of specific binding to the analyte biomarker. A ligand according to the invention may be labelled with a detectable marker, such as a luminescent, fluorescent or radioactive marker; alternatively or additionally a ligand according to the invention may be labelled with an affinity tag, e.g. a biotin, avidin, streptavidin or His (e.g. hexa-His) tag.

A biosensor may comprise the analyte biomarker or a structural/shape mimic thereof capable of specific binding to an antibody against the analyte biomarker. Also provided is an array comprising a ligand or mimic as described herein.

Also provided is the use of one or more ligands as described herein, which may be naturally occurring or chemically synthesised, and is suitably a peptide, antibody or fragment thereof, aptamer or oligonucleotide, or the use of a biosensor, or an array, or a kit to detect and/or quantify the analyte. In these uses, the detection and/or quantification can be performed on a biological sample such as from the group consisting of CSF, whole blood, blood serum, plasma, urine, saliva, or other bodily fluid, breath, e.g. as condensed breath, or an extract or purification therefrom, or dilution thereof.

Uses of diagnostic or monitoring kits are provided for performing methods of the invention. Such kits will suitably comprise a ligand, for detection and/or quantification of the analyte biomarker, and/or a biosensor, and/or an array as described herein, optionally together with instructions for use of the kit.

A further aspect of the invention is a use of a kit for monitoring or diagnosing schizophrenia or other psychotic disorder, comprising a biosensor capable of detecting and/or quantifying the panel of biomarkers as defined herein.

Biomarkers for schizophrenia or other psychotic disorders are essential targets for discovery of novel targets and drug molecules that retard or halt progression of the disorder. As the level of the analyte biomarker is indicative of disorder and of drug response, the biomarker is useful for identification of novel therapeutic compounds in *in vitro* and/or *in vivo* assays. Biomarkers can be employed in methods for screening for compounds that modulate the activity of the analyte.

Thus, in a further aspect, there is provided the use of a ligand, as described, which can be a peptide, antibody or fragment thereof or aptamer or oligonucleotide; or the use of a biosensor, or an array; or a kit, to identify a substance capable of promoting and/or of suppressing the generation of the biomarker.

Also there is provided a method of identifying a substance capable of promoting or suppressing the generation of the analyte in a subject, comprising administering a test substance to a subject animal and detecting and/or quantifying the level of the analyte biomarker present in a test sample from the subject.

### BRIEF DESCRIPTION OF THE FIGURES

**Figure 1** describes 30 significant biomarkers which were found to provide a sensitive diagnostic for schizophrenia.
**Figure 2** describes 21 significant biomarkers which demonstrated effective diagnosis of bipolar disorder.
**Figure 3** describes the reproducibility of schizophrenia diagnosis and effect of the analyte biomarker in schizophrenia patients across the 5 cohorts for the 30 biomarkers listed in Figure 1.
**Figure 4** describes the reproducibility of schizophrenia diagnosis and effect of the analyte biomarker in schizophrenia patients across the 5 cohorts for the 21 biomarkers listed in Figure 2.
**Figure 5** describes a statistical analysis which indicates the optimum number of biomarkers in the panel of the invention.

### DETAILED DESCRIPTION OF THE INVENTION

The term "biomarker" means a distinctive biological or biologically derived indicator of a process, event, or condition. Analyte biomarkers can be used in methods of diagnosis, e.g. clinical screening, and prognosis assessment and in monitoring the results of therapy, identifying patients most likely to respond to a particular therapeutic treatment, drug screening and development. Biomarkers and uses thereof are valuable for identification of new drug treatments and for discovery of new targets for drug treatment.

It will be readily apparent to the skilled person that the analytes listed herein are known and have been described in the literature (WO 2009/077763).

According to a first aspect of the invention, there is provided the use of Alpha 1 Antitrypsin, Apolipoprotein A1, Cortisol, Ferritin and IgA as a specific panel of biomarkers for schizophrenia, or predisposition thereto.

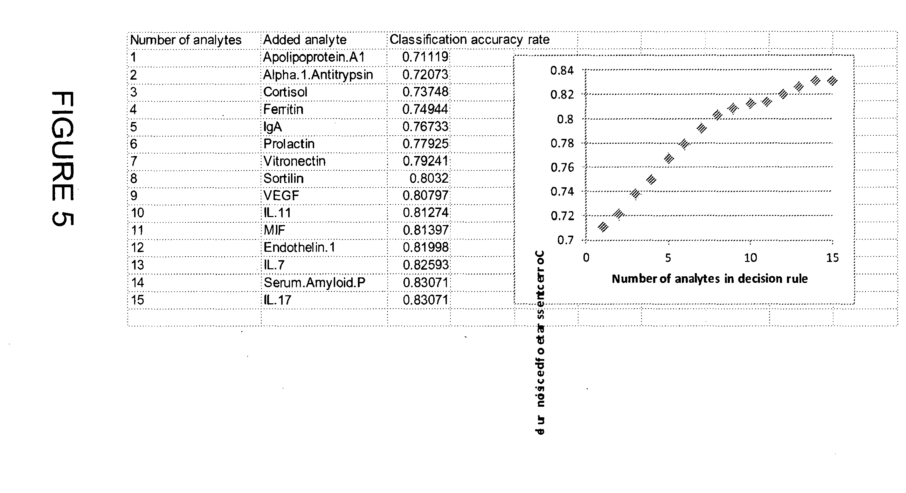

Results of a statistical analysis with a known algorithm are shown in Figure 5 wherein it can be seen that the most predictive analyte (Alpha 1 Antitrypsin) achieved a classification accuracy rate of 0.71119. The next most predictive analyte biomarkers were then added to the algorithm to improve statistical performance until it can no longer be improved. It can be seen from the results shown in Figure 5 that a panel of the 5 most predictive biomarkers achieved a correctness rate of greater than 0.75. The results of the analysis shown in Figure 5 therefore demonstrate that the specific panel of the invention provides a specific and sensitive diagnosis of schizophrenia with a classification accuracy rate in excess of 0.75.

In one embodiment of the first aspect of the invention, the panel additionally comprises Prolactin, Vitronectin, Sortilin, VEGF, IL-11, MIF, Endothelin-1, IL-7, Serum Amyloid P and IL-17. This embodiment comprises the remaining 10 analyte biomarkers which were used in the study shown in Figure 5 which indicates that after the addition of 15 markers the performance could no longer be improved. Thus, according to a further aspect of the invention, there is provided the use of Alpha 1 Antitrypsin, Apolipoprotein A1, Cortisol, Ferritin, IgA, Prolactin, Vitronectin, Sortilin, VEGF, IL-11, MIF, Endothelin-1, IL-7, Serum Amyloid P and IL-17 as a specific panel of biomarkers for schizophrenia, or predisposition thereto.

In an alternative embodiment of the first aspect of the invention, the panel additionally comprises Brain Derived Neurotrophic Factor (BDNF), Haptoglobin, ICAM-1, MIF, Serum Amyloid P, Sortilin, Testosterone, Thyroid Stimulating Hormone (TSH), TNF RII, Transferrin and Vitronectin. Thus, according to a further aspect of the invention, there is provided the use of Alpha 1 Antitrypsin, Apolipoprotein A1, Cortisol, Ferritin, IgA, Brain Derived Neurotrophic Factor (BDNF), Haptoglobin, ICAM-1, MIF, Serum Amyloid P, Sortilin, Testosterone, Thyroid Stimulating Hormone (TSH), TNF RII, Transferrin and Vitronectin as a specific panel of biomarkers for schizophrenia or predisposition thereto.

In a further embodiment of the first aspect of the invention, the panel additionally comprises Apolipoprotein A2, Beta-2 Microglobulin, EGF receptor, Alpha-2-HS-glycoprotein (Fetuin A), IgM, IL-6 receptor, MDC, TRAIL R3 and VEGF. Thus, according to a further aspect of the invention, there is provided the use of Alpha 1 Antitrypsin, Apolipoprotein A1, Cortisol, Ferritin, IgA, Brain Derived Neurotrophic Factor (BDNF), Haptoglobin, ICAM-1, MIF, Serum Amyloid P, Sortilin, Testosterone, Thyroid Stimulating Hormone (TSH), TNF RII, Transferrin, Vitronectin, Apolipoprotein A2, Beta-2 Microglobulin, EGF receptor, Alpha-2-HS-glycoprotein (Fetuin A), IgM, IL-6 receptor, MDC, TRAIL R3 and VEGF as a specific panel of biomarkers for schizophrenia, or predisposition thereto.

In a further embodiment of the first aspect of the invention, the panel additionally comprises Cancer antigen 125, Kidney injury molecule 1, MCP-2, Apolipoprotein H, Carcinoembryonic antigen (CEA), Complement 3, IL-10, Prolactin, Tissue Inhibitor of Metalloprotease-1 (TIMP 1), Betacellulin, Connective Tissue Growth Factor (CTGF), Endothelin-1, Luteinizing hormone (LH), MIP-1 alpha, Prostatic Acid Phosphatase, Apolipoprotein B, Apolipoprotein CI, IL-11, IL-17, Thrombopoeitin, Calbindin, CD5L, Follicle Stimulating Hormone (FSH), IL-7, MMP-2 and Peptide YY (PYY). Thus, according to a further aspect of the invention, there is provided the use of Alpha 1 Antitrypsin, Apolipoprotein A1, Cortisol, Ferritin, IgA, Brain Derived Neurotrophic Factor (BDNF), Haptoglobin, ICAM-1, MIF, Serum Amyloid P, Sortilin, Testosterone, Thyroid Stimulating Hormone (TSH), TNF RII, Transferrin, Vitronectin, Apolipoprotein A2, Beta-2 Microglobulin, EGF receptor, Alpha-2-HS-glycoprotein (Fetuin A), IgM, IL-6 receptor, MDC, TRAIL R3, VEGF, Cancer antigen 125, Kidney injury molecule 1, MCP-2, Apolipoprotein H, Carcinoembryonic antigen (CEA), Complement 3, IL-10, Prolactin, Tissue Inhibitor of Metalloprotease-1 (TIMP 1), Betacellulin, Connective Tissue Growth Factor (CTGF), Endothelin-1, Luteinizing hormone (LH), MIP-1 alpha, Prostatic Acid Phosphatase, Apolipoprotein B, Apolipoprotein CI, IL-11, IL-17, Thrombopoeitin, Calbindin, CD5L, Follicle Stimulating Hormone (FSH), IL-7, MMP-2 and Peptide YY (PYY) as a specific panel of biomarkers for schizophrenia, or predisposition thereto.

In one embodiment of the first aspect of the invention, said diagnosis comprises differential diagnosis of schizophrenia or other psychotic disorder over bipolar disorder.

It is further disclosed the use of Cancer antigen 125, MCP-2, Alpha 1 Antitrypsin, Apolipoprotein A1, Apolipoprotein H, Carcinoembryonic antigen (CEA), Complement 3, Cortisol, Ferritin, Haptoglobin, ICAM-1, IL-10, MIF, Prolactin, Serum Amyloid P, Tissue Inhibitor of Metalloprotease-1 (TIMP 1), TNF RII, TRAIL R3, VEGF, Brain Derived Neurotrophic Factor (BDNF), Beta-2 Microglobulin, Betacellulin, Connective Tissue Growth Factor (CTGF), Endothelin-1, Alpha-2-HS-glycoprotein (Fetuin A), IgA, Luteinizing hormone (LH), MDC, Prostatic Acid Phosphatase, Sortilin, Apolipoprotein A2, IL-17, Thrombopoeitin, Calbindin, CD5L, Follicle Stimulating Hormone (FSH), IL-6 receptor, IL-7, MMP-2, Peptide YY (PYY) and Transferrin as a specific panel of analyte biomarkers for schizophrenia or other psychotic disorder, or predisposition thereto.

It is further disclosed the use of Alpha 1 Antitrypsin, Apolipoprotein A1, Apolipoprotein H, Carcinoembryonic antigen (CEA), Complement 3, Cortisol, Ferritin, Haptoglobin, ICAM-1, IL-10, MIF, Prolactin, Serum Amyloid P, Tissue Inhibitor of Metalloprotease-1 (TIMP 1), TNF RII, TRAIL R3, VEGF, Brain Derived Neurotrophic Factor (BDNF), Beta-2 Microglobulin, Betacellulin, Connective Tissue Growth Factor (CTGF), Endothelin-1, Alpha-2-HS-glycoprotein (Fetuin A), IgA, Luteinizing hormone (LH), MDC, MIP-1 alpha, Prostatic Acid Phosphatase, Sortilin, Testosterone and Thrombopoeitin as a specific panel of analyte biomarkers for schizophrenia or other psychotic disorder, or predisposition thereto.

The invention relates to the identification of a specific and unique pattern of serum analyte biomarkers which can be used for diagnosis of schizophrenia in a specific and sensitive manner. Using an immunoassay platform (HumanMAP®, Rules Based Medicine, Austin, Texas), between 147 and 187 analytes were measured in the serum of subjects recruited at five clinical centres (henceforth called cohort 1, 2, 3, 4 and 5) as well as the US military (cohort 6). Cohorts 1-5 comprised schizophrenia patients classified as the paranoid subtype (295.30), cohort 6 contained pre-symptomatic subjects, later diagnosed with bipolar disorder (final classification 296.00-296.06; 296.40-296.7 and 296.89). All cohorts comprised control subjects who were matched for gender, age and social demographics. Patients from cohorts 1, 2, 5 and 6 were drug naïve. Most patients from cohort 3 and all patients from cohort 4 were either drug naïve or had either been off medication for at least six weeks prior to sample collection. The remaining patients from cohort 3 (n=12) were medicated at the time of sample collection.

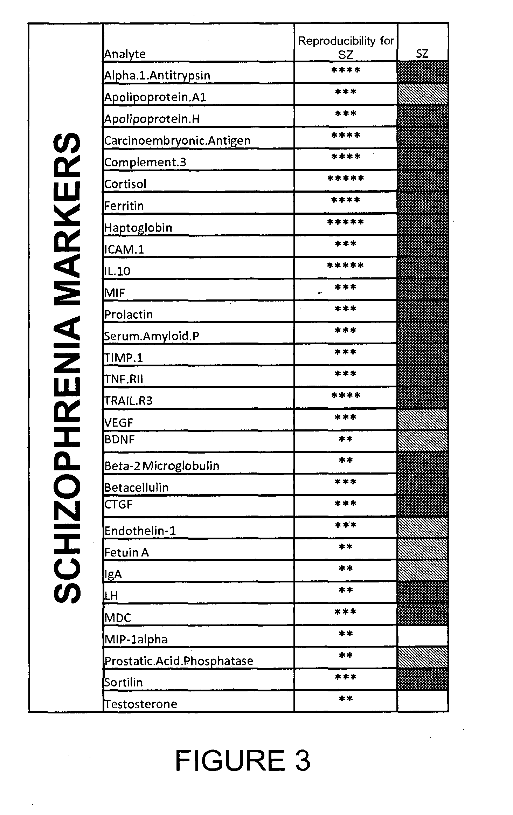

The identification of the biomarker panel consisted of two stages. In the first stage, the differences between schizophrenia patients and controls were assessed in each clinical centre using parametric t-tests (two tailed). Results were compared against non-parametric Wilcoxon rank sum tests which yielded very similar results. All measured analytes were then ranked based on the reproducibility of their significance across centers. The 30 analytes with the highest rank were then chosen to represent the schizophrenia specific part of the biomarker panel (Figure 3). Markers that were measured with high variability and that could not be detected reproducibly across centers were excluded from the ranking process. Thus, it is further disclosed the use of Alpha 1 Antitrypsin, Apolipoprotein A1, Apolipoprotein H, Carcinoembryonic antigen (CEA), Complement 3, Cortisol, Ferritin, Haptoglobin, ICAM-1, IL-10, MIF, Prolactin, Serum Amyloid P, Tissue Inhibitor of Metalloprotease-1 (TIMP 1), TNF RII, TRAIL R3, VEGF, Brain Derived Neurotrophic Factor (BDNF), Beta-2 Microglobulin, Betacellulin, Connective Tissue Growth Factor (CTGF), Endothelin-1, Alpha-2-HS-glycoprotein (Fetuin A), IgA, Luteinizing hormone (LH), MDC, MIP-1 alpha, Prostatic Acid Phosphatase, Sortilin and Testosterone as a specific panel of analyte biomarkers for schizophrenia, or predisposition thereto.

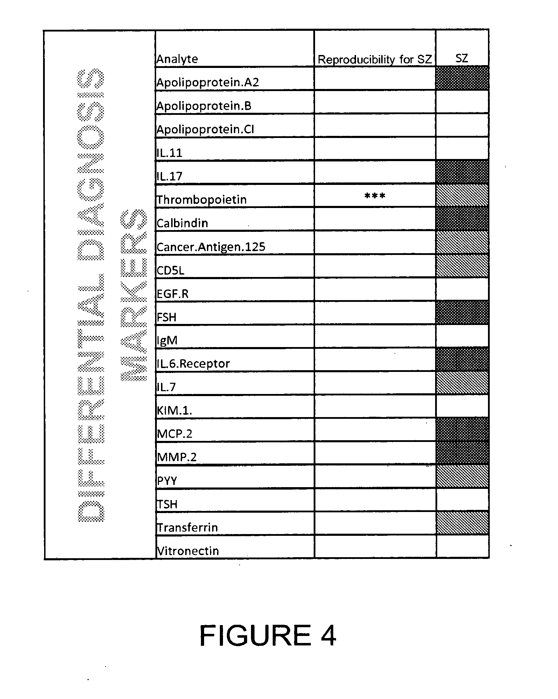

In the second stage of the analysis, the biomarker panel was extended to include markers specific for bipolar disorder. Therefore, the differences between pre-symptomatic bipolar disorder patients and matched controls were assessed in cohort 6 using parametric t-tests. All 21 significant analytes were added to the biomarker panel as differential diagnosis markers (Figure 4).

The biomarker panel includes a total of 51 analytes. Thus, according to a further aspect of the invention there is provided the use of Cancer antigen 125, Kidney injury molecule 1, MCP-2, Alpha 1 Antitrypsin, Apolipoprotein A1, Apolipoprotein H, Carcinoembryonic antigen (CEA), Complement 3, Cortisol, Ferritin, Haptoglobin, ICAM-1, IL-10, MIF, Prolactin, Serum Amyloid P, Tissue Inhibitor of Metalloprotease-1 (TIMP 1), TNF RII, TRAIL R3, VEGF, Brain Derived Neurotrophic Factor (BDNF), Beta-2 Microglobulin, Betacellulin, Connective Tissue Growth Factor (CTGF), Endothelin-1, Alpha-2-HS-glycoprotein (Fetuin A), IgA, Luteinizing hormone (LH), MDC, MIP-1 alpha, Prostatic Acid Phosphatase, Sortilin, Testosterone, Apolipoprotein A2, Apolipoprotein B, Apolipoprotein CI, IL-11, IL-17, Thrombopoeitin, Calbindin, CD5L, EGF receptor, Follicle Stimulating Hormone (FSH), IgM, IL-6 receptor, IL-7, MMP-2, Peptide YY (PYY), Thyroid Stimulating Hormone (TSH), Transferrin and Vitronectin as a specific panel of analyte biomarkers for differential diagnosis of schizophrenia over bipolar disorder.

The schizophrenia part of the biomarker panel (i.e. the panel of 30 biomarkers) can distinguish first onset, recent onset and drug treated patients from healthy controls with high sensitivity and specificity. A classification algorithm built on a subset of these markers can blindly classify patients and controls (when trained on cohort one) from cohort 2 to 5 with an average sensitivity 77% and an average specificity of 78%.

The data presented herein clearly demonstrates the ability of the 30 serum biomarkers to be capable of classifying schizophrenia patients with high sensitivity and specificity compared to control subjects and the additional 21 serum biomarkers are additionally capable of classifying patients with other neuropsychiatric disorders, such as bipolar disorder.

Therefore, the specific panel of 51 serum biomarkers provided by this aspect of the invention is a sensitive, specific and differential predictor for the presence of schizophrenia or other psychotic disorder.

It will be appreciated that the specific panel of 51 serum biomarkers constitutes a key aspect of the invention. However, it will be appreciated that it could be envisaged to omit one or more of the serum biomarkers in the specific panel and still achieve the object of the invention to provide a sensitive and specific predictor for the presence of schizophrenia or other psychotic disorder. Thus, any one or more of the following single or combined embodiments may apply: In one embodiment of any of the aforementioned aspects of the invention, the analyte biomarker is other than Cancer antigen 125. In one embodiment of any of the aforementioned aspects of the invention, the analyte biomarker is other than Kidney injury molecule 1. In one embodiment of any of the aforementioned aspects of the invention, the analyte biomarker is other than MCP-2. In one embodiment of any of the aforementioned aspects of the invention, the analyte biomarker is other than Alpha 1 Antitrypsin. In one embodiment of any of the aforementioned aspects of the invention, the analyte biomarker is other than Apolipoprotein A1. In one embodiment of any of the aforementioned aspects of the invention, the analyte biomarker is other than Apolipoprotein H. In one embodiment of any of the aforementioned aspects of the invention, the analyte biomarker is other than Carcinoembryonic antigen (CEA). In one embodiment of any of the aforementioned aspects of the invention, the analyte biomarker is other than Complement 3. In one embodiment of any of the aforementioned aspects of the invention, the analyte biomarker is other than Cortisol. In one embodiment of any of the aforementioned aspects of the invention, the analyte biomarker is other than Ferritin. In one embodiment of any of the aforementioned aspects of the invention, the analyte biomarker is other than Haptoglobin. In one embodiment of any of the aforementioned aspects of the invention, the analyte biomarker is other than ICAM-1. In one embodiment of any of the aforementioned aspects of the invention, the analyte biomarker is other than IL-10. In one embodiment of any of the aforementioned aspects of the invention, the analyte biomarker is other than MIF. In one embodiment of any of the aforementioned aspects of the invention, the analyte biomarker is other than Prolactin. In one embodiment of any of the aforementioned aspects of the invention, the analyte biomarker is other than Serum Amyloid P. In one embodiment of any of the aforementioned aspects of the invention, the analyte biomarker is other than Tissue Inhibitor of Metalloprotease-1 (TIMP 1). In one embodiment of any of the aforementioned aspects of the invention, the analyte biomarker is other than TNF RII. In one embodiment of any of the aforementioned aspects of the invention, the analyte biomarker is other than TRAIL R3. In one embodiment of any of the aforementioned aspects of the invention, the analyte biomarker is other than VEGF. In one embodiment of any of the aforementioned aspects of the invention, the analyte biomarker is other than Brain Derived Neurotrophic Factor (BDNF). In one embodiment of any of the aforementioned aspects of the invention, the analyte biomarker is other than Beta-2 Microglobulin. In one embodiment of any of the aforementioned aspects of the invention, the analyte biomarker is other than Betacellulin. In one embodiment of any of the aforementioned aspects of the invention, the analyte biomarker is other than Connective Tissue Growth Factor (CTGF). In one embodiment of any of the aforementioned aspects of the invention, the analyte biomarker is other than Endothelin-1. In one embodiment of any of the aforementioned aspects of the invention, the analyte biomarker is other than Alpha-2-HS-glycoprotein (Fetuin A). In one embodiment of any of the aforementioned aspects of the invention, the analyte biomarker is other than IgA. In one embodiment of any of the aforementioned aspects of the invention, the analyte biomarker is other than Luteinizing hormone (LH). In one embodiment of any of the aforementioned aspects of the invention, the analyte biomarker is other than MDC. In one embodiment of any of the aforementioned aspects of the invention, the analyte biomarker is other than MIP-1 alpha. In one embodiment of any of the aforementioned aspects of the invention, the analyte biomarker is other than Prostatic Acid Phosphatase. In one embodiment of any of the aforementioned aspects of the invention, the analyte biomarker is other than Sortilin. In one embodiment of any of the aforementioned aspects of the invention, the analyte biomarker is other than Testosterone. In one embodiment of any of the aforementioned aspects of the invention, the analyte biomarker is other than Apolipoprotein A2. In one embodiment of any of the aforementioned aspects of the invention, the analyte biomarker is other than Apolipoprotein B. In one embodiment of any of the aforementioned aspects of the invention, the analyte biomarker is other than Apolipoprotein CI. In one embodiment of any of the aforementioned aspects of the invention, the analyte biomarker is other than IL-11. In one embodiment of any of the aforementioned aspects of the invention, the analyte biomarker is other than IL-17. In one embodiment of any of the aforementioned aspects of the invention, the analyte biomarker is other than Thrombopoeitin. In one embodiment of any of the aforementioned aspects of the invention, the analyte biomarker is other than Calbindin. In one embodiment of any of the aforementioned aspects of the invention, the analyte biomarker is other than CD5L. In one embodiment of any of the aforementioned aspects of the invention, the analyte biomarker is other than EGF receptor. In one embodiment of any of the aforementioned aspects of the invention, the analyte biomarker is other than Follicle Stimulating Hormone (FSH). In one embodiment of any of the aforementioned aspects of the invention, the analyte biomarker is other than IgM. In one embodiment of any of the aforementioned aspects of the invention, the analyte biomarker is other than IL-6 receptor. In one embodiment of any of the aforementioned aspects of the invention, the analyte biomarker is other than IL-7. In one embodiment of any of the aforementioned aspects of the invention, the analyte biomarker is other than MMP-2. In one embodiment of any of the aforementioned aspects of the invention, the analyte biomarker is other than Peptide YY (PYY). In one embodiment of any of the aforementioned aspects of the invention, the analyte biomarker is other than Thyroid Stimulating Hormone (TSH). In one embodiment of any of the aforementioned aspects of the invention, the analyte biomarker is other than Transferrin. In one embodiment of any of the aforementioned aspects of the invention, the analyte biomarker is other than Vitronectin.

References herein to "other psychotic disorder" relate to any appropriate psychotic disorder according to DSM-IV Diagnostic and Statistical Manual of Mental Disorders, 4th edition, American Psychiatric Assoc, Washington, D.C., 2000. In one particular embodiment, the other psychotic disorder is a psychotic disorder related to schizophrenia. Examples of psychotic disorders related to schizophrenia include brief psychotic disorder delusional disorder, psychotic disorder due to a general medical condition, schizoeffective disorder, schizophreniform disorder, and substance-induced psychotic disorder.

As used herein, the term "biosensor" means anything capable of detecting the presence of the biomarker. Examples of biosensors are described herein.

Biosensors may comprise a ligand or ligands, as described herein, capable of specific binding to the analyte biomarker. Such biosensors are useful in detecting and/or quantifying an analyte of the invention.

Diagnostic kits for the diagnosis and monitoring of schizophrenia or other psychotic disorder are described herein. In one embodiment, the kits additionally contain a biosensor capable of detecting and/or quantifying an analyte biomarker.

Monitoring methods can be used to monitor onset, progression, stabilisation, amelioration and/or remission.

In methods of diagnosing or monitoring according to the invention, detecting and/or quantifying the analyte biomarker in a biological sample from a test subject may be performed on two or more occasions. Comparisons may be made between the level of biomarker in samples taken on two or more occasions. Assessment of any change in the level of the analyte biomarker in samples taken on two or more occasions may be performed. Modulation of the analyte biomarker level is useful as an indicator of the state of schizophrenia or other psychotic disorder or predisposition thereto. An increase in the level of the biomarker, over time is indicative of onset or progression, i.e. worsening of this disorder, whereas a decrease in the level of the analyte biomarker indicates amelioration or remission of the disorder, or vice versa.

A method of diagnosis or monitoring according to the invention may comprise quantifying the analyte biomarker in a test biological sample from a test subject and comparing the level of the analyte present in said test sample with one or more controls.

The control can be one or more control(s) selected from the group consisting of: the level of biomarker analyte found in a normal control sample from a normal subject, a normal biomarker analyte level; a normal biomarker analyte range, the level in a sample from a subject with schizophrenia or other psychotic disorder, or a diagnosed predisposition thereto; schizophrenia or other psychotic disorder biomarker analyte level, or schizophrenia or other psychotic disorder biomarker analyte range.

There is provided a method of diagnosing schizophrenia or other psychotic disorder, or predisposition thereto, which comprises:
(a) quantifying the amount of the analyte biomarker in a test biological sample; and
(b) comparing the amount of said analyte in said test sample with the amount present in a normal control biological sample from a normal subject.

A higher level of the analyte biomarker in the test sample relative to the level in the normal control is indicative of the presence of schizophrenia or other psychotic disorder, or predisposition thereto; an equivalent or lower level of the analyte in the test sample relative to the normal control is indicative of absence of schizophrenia or other psychotic disorder and/or absence of a predisposition thereto.

The term "diagnosis" as used herein encompasses identification, confirmation, and/or characterisation of schizophrenia or other psychotic disorder, or predisposition thereto. By predisposition it is meant that a subject does not currently present with the disorder, but is liable to be affected by the disorder in time. Methods of monitoring and of diagnosis according to the invention are useful to confirm the existence of a disorder, or predisposition thereto; to monitor development of the disorder by assessing onset and progression, or to assess amelioration or regression of the disorder. Methods of monitoring and of diagnosis are also useful in methods for assessment of clinical screening, prognosis, choice of therapy, evaluation of therapeutic benefit, i.e. for drug screening and drug development.

Efficient diagnosis and monitoring methods provide very powerful "patient solutions" with the potential for improved prognosis, by establishing the correct diagnosis, allowing rapid identification of the most appropriate treatment (thus lessening unnecessary exposure to harmful drug side effects), reducing relapse rates.

Also provided is a method of monitoring efficacy of a therapy for schizophrenia or other psychotic disorder in a subject having such a disorder, suspected of having such a disorder, or of being predisposed thereto, comprising detecting and/or quantifying the analyte present in a biological sample from said subject. In monitoring methods, test samples may be taken on two or more occasions. The method may further comprise comparing the level of the biomarker(s) present in the test sample with one or more control(s) and/or with one or more previous test sample(s) taken earlier from the same test subject, e.g. prior to commencement of therapy, and/or from the same test subject at an earlier stage of therapy. The method may comprise detecting a change in the level of the biomarker(s) in test samples taken on different occasions.

Provided is a method for monitoring efficacy of therapy for schizophrenia or other psychotic disorder in a subject, comprising:
(a) quantifying the amount of the analyte biomarker; and
(b) comparing the amount of said analyte in said test sample with the amount present in one or more control(s) and/or one or more previous test sample(s) taken at an earlier time from the same test subject.

A decrease in the level of the analyte biomarker in the test sample relative to the level in a previous test sample taken earlier from the same test subject is indicative of a beneficial effect, e.g. stabilisation or improvement, of said therapy on the disorder, suspected disorder or predisposition thereto.

Methods for monitoring efficacy of a therapy can be used to monitor the therapeutic effectiveness of existing therapies and new therapies in human subjects and in non-human animals (e.g. in animal models). These monitoring methods can be incorporated into screens for new drug substances and combinations of substances.

Suitably, the time elapsed between taking samples from a subject undergoing diagnosis or monitoring will be 3 days, 5 days, a week, two weeks, a month, 2 months, 3 months, 6 or 12 months. Samples may be taken prior to and/or during and/or following an anti-psychotic therapy. Samples can be taken at intervals over the remaining life, or a part thereof, of a subject.

The term "detecting" as used herein means confirming the presence of the analyte biomarker present in the sample. Quantifying the amount of the biomarker present in a sample may include determining the concentration of the analyte biomarker present in the sample. Detecting and/or quantifying may be performed directly on the sample, or indirectly on an extract therefrom, or on a dilution thereof.

In alternative aspects the presence of the analyte biomarker is assessed by detecting and/or quantifying antibody or fragments thereof capable of specific binding to the biomarker that are generated by the subject's body in response to the analyte and thus are present in a biological sample from a subject having schizophrenia or other psychotic disorder or a predisposition thereto.

Detecting and/or quantifying can be performed by any method suitable to identify the presence and/or amount of a specific protein in a biological sample from a patient or a purification or extract of a biological sample or a dilution thereof. In methods of the invention, quantifying may be performed by measuring the concentration of the analyte biomarker in the sample or samples. Biological samples that may be tested in a method of the invention include cerebrospinal fluid (CSF), whole blood, blood serum, plasma, urine, saliva, or other bodily fluid (stool, tear fluid, synovial fluid, sputum), breath, e.g. as condensed breath, or an extract or purification therefrom, or dilution thereof. Biological samples also include tissue homogenates, tissue sections and biopsy specimens from a live subject, or taken post-mortem. The samples can be prepared, for example where appropriate diluted or concentrated, and stored in the usual manner.

Detection and/or quantification of analyte biomarkers may be performed by detection of the analyte biomarker or of a fragment thereof, e.g. a fragment with C-terminal truncation, or with N-terminal truncation. Fragments are suitably greater than 4 amino acids in length, for example 5, 6, 7, 8, 9, 10, 11, 12, 13, 14, 15, 16, 17, 18, 19, or 20 amino acids in length.

The biomarker may be directly detected, e.g. by SELDI or MALDI-TOF. Alternatively, the biomarker may be detected directly or indirectly via interaction with a ligand or ligands such as an antibody or a biomarker-binding fragment thereof, or other peptide, or ligand, e.g. aptamer, or oligonucleotide, capable of specifically binding the biomarker. The ligand may possess a detectable label, such as a luminescent, fluorescent or radioactive label, and/or an affinity tag.

For example, detecting and/or quantifying can be performed by one or more method(s) selected from the group consisting of: SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC and other LC or LC MS-based techniques. Appropriate LC MS techniques include ICAT® (Applied Biosystems, CA, USA), or iTRAQ® (Applied Biosystems, CA, USA). Liquid chromatography (e.g. high pressure liquid chromatography (HPLC) or low pressure liquid chromatography (LPLC)), thin-layer chromatography, NMR (nuclear magnetic resonance) spectroscopy could also be used.

Methods of diagnosing or monitoring may comprise analysing a sample of cerebrospinal fluid (CSF) by SELDI TOF or MALDI TOF to detect the presence or level of the analyte biomarker. These methods are also suitable for clinical screening, prognosis, monitoring the results of therapy, identifying patients most likely to respond to a particular therapeutic treatment, for drug screening and development, and identification of new targets for drug treatment.

Detecting and/or quantifying the analyte biomarkers may be performed using an immunological method, involving an antibody, or a fragment thereof capable of specific binding to the analyte biomarker. Suitable immunological methods include sandwich immunoassays, such as sandwich ELISA, in which the detection of the analyte biomarkers is performed using two antibodies which recognize different epitopes on a analyte biomarker; radioimmunoassays (RIA), direct, indirect or competitive enzyme linked immunosorbent assays (ELISA), enzyme immunoassays (EIA), Fluorescence immunoassays (FIA), western blotting, immunoprecipitation and any particle-based immunoassay (e.g. using gold, silver, or latex particles, magnetic particles, or Q-dots). Immunological methods may be performed, for example, in microtitre plate or strip format.

Immunological methods may be based, for example, on any of the following methods.

Immunoprecipitation is the simplest immunoassay method; this measures the quantity of precipitate, which forms after the reagent antibody has incubated with the sample and reacted with the target antigen present therein to form an insoluble aggregate. Immunoprecipitation reactions may be qualitative or quantitative.

In particle immunoassays, several antibodies are linked to the particle, and the particle is able to bind many antigen molecules simultaneously. This greatly accelerates the speed of the visible reaction. This allows rapid and sensitive detection of the biomarker.

In immunonephelometry, the interaction of an antibody and target antigen on the biomarker results in the formation of immune complexes that are too small to precipitate. However, these complexes will scatter incident light and this can be measured using a nephelometer. The antigen, i.e. biomarker, concentration can be determined within minutes of the reaction.

Radioimmunoassay (RIA) methods employ radioactive isotopes such as I¹²⁵ to label either the antigen or antibody. The isotope used emits gamma rays, which are usually measured following removal of unbound (free) radiolabel. The major advantages of RIA, compared with other immunoassays, are higher sensitivity, easy signal detection, and well-established, rapid assays. The major disadvantages are the health and safety risks posed by the use of radiation and the time and expense associated with maintaining a licensed radiation safety and disposal program. For this reason, RIA has been largely replaced in routine clinical laboratory practice by enzyme immunoassays.

Enzyme (EIA) immunoassays were developed as an alternative to radioimmunoassays (RIA). These methods use an enzyme to label either the antibody or target antigen. The sensitivity of EIA approaches that for RIA, without the danger posed by radioactive isotopes. One of the most widely used EIA methods for detection is the enzyme-linked immunosorbent assay (ELISA). ELISA methods may use two antibodies one of which is specific for the target antigen and the other of which is coupled to an enzyme, addition of the substrate for the enzyme results in production of a chemiluminescent or fluorescent signal.

Fluorescent immunoassay (FIA) refers to immunoassays which utilize a fluorescent label or an enzyme label which acts on the substrate to form a fluorescent product. Fluorescent measurements are inherently more sensitive than colorimetric (spectrophotometric) measurements. Therefore, FIA methods have greater analytical sensitivity than EIA methods, which employ absorbance (optical density) measurement.

Chemiluminescent immunoassays utilize a chemiluminescent label, which produces light when excited by chemical energy; the emissions are measured using a light detector.

Immunological methods can thus be performed using well-known methods. Any direct (e.g., using a sensor chip) or indirect procedure may be used in the detection of analyte biomarkers.

The Biotin-Avidin or Biotin-Streptavidin systems are generic labelling systems that can be adapted for use in immunological methods of the invention. One binding partner (hapten, antigen, ligand, aptamer, antibody, enzyme etc) is labelled with biotin and the other partner (surface, e.g. well, bead, sensor etc) is labelled with avidin or streptavidin. This is conventional technology for immunoassays, gene probe assays and (bio)sensors, but is an indirect immobilisation route rather than a direct one. For example a biotinylated ligand (e.g. antibody or aptamer) specific for an analyte biomarker of the invention may be immobilised on an avidin or streptavidin surface, the immobilised ligand may then be exposed to a sample containing or suspected of containing the analyte biomarker in order to detect and/or quantify an analyte biomarker. Detection and/or quantification of the immobilised antigen may then be performed by an immunological method as described herein.

The term "antibody" as used herein includes, but is not limited to: polyclonal, monoclonal, bispecific, humanised or chimeric antibodies, single chain antibodies, Fab fragments and F(ab')₂ fragments, fragments produced by a Fab expression library, anti-idiotypic (anti-Id) antibodies and epitope-binding fragments of any of the above. The term "antibody" as used herein also refers to immunoglobulin molecules and immunologically-active portions of immunoglobulin molecules, i.e., molecules that contain an antigen binding site that specifically binds an antigen. The immunoglobulin molecules can be of any class (e. g., IgG, IgE, IgM, IgD and IgA) or subclass of immunoglobulin molecule.

The identification of key biomarkers specific to a disease is central to integration of diagnostic procedures and therapeutic regimes. Using predictive biomarkers appropriate diagnostic tools such as biosensors can be developed; accordingly, in methods and uses of the invention, detecting and quantifying can be performed using a biosensor, microanalytical system, microengineered system, microseparation system, immunochromatography system or other suitable analytical devices. The biosensor may incorporate an immunological method for detection of the biomarker(s), electrical, thermal, magnetic, optical (e.g. hologram) or acoustic technologies. Using such biosensors, it is possible to detect the target biomarker(s) at the anticipated concentrations found in biological samples.

Thus, according to a further aspect there is provided an apparatus for diagnosing or monitoring schizophrenia or other psychotic disorders which comprises a biosensor, microanalytical, microengineered, microseparation and/or immunochromatography system configured to detect and/or quantify any of the biomarkers defined herein.

The biomarker(s) can be detected using a biosensor incorporating technologies based on "smart" holograms, or high frequency acoustic systems, such systems are particularly amenable to "bar code" or array configurations.

In smart hologram sensors (Smart Holograms Ltd, Cambridge, UK), a holographic image is stored in a thin polymer film that is sensitised to react specifically with the biomarker. On exposure, the biomarker reacts with the polymer leading to an alteration in the image displayed by the hologram. The test result read-out can be a change in the optical brightness, image, colour and/or position of the image. For qualitative and semi-quantitative applications, a sensor hologram can be read by eye, thus removing the need for detection equipment. A simple colour sensor can be used to read the signal when quantitative measurements are required. Opacity or colour of the sample does not interfere with operation of the sensor. The format of the sensor allows multiplexing for simultaneous detection of several substances. Reversible and irreversible sensors can be designed to meet different requirements, and continuous monitoring of a particular biomarker of interest is feasible.

Suitably, biosensors for detection of one or more biomarkers combine biomolecular recognition with appropriate means to convert detection of the presence, or quantitation, of the biomarker in the sample into a signal. Biosensors can be adapted for "alternate site" diagnostic testing, e.g. in the ward, outpatients' department, surgery, home, field and workplace.

Biosensors to detect one or more biomarkers include acoustic, plasmon resonance, holographic and microengineered sensors. Imprinted recognition elements, thin film transistor technology, magnetic acoustic resonator devices and other novel acousto-electrical systems may be employed in biosensors for detection of the one or more biomarkers.

Methods involving detection and/or quantification of one or more analyte biomarkers can be performed on bench-top instruments, or can be incorporated onto disposable, diagnostic or monitoring platforms that can be used in a non-laboratory environment, e.g. in the physician's office or at the patient's bedside. Suitable biosensors for performing methods of the invention include "credit" cards with optical or acoustic readers. Biosensors can be configured to allow the data collected to be electronically transmitted to the physician for interpretation and thus can form the basis for e-neuromedicine.

Any suitable animal may be used as a subject non-human animal, for example a non-human primate, horse, cow, pig, goat, sheep, dog, cat, fish, rodent, e.g. guinea pig, rat or mouse; insect (e.g. *Drosophila),* amphibian (e.g. *Xenopus*) or C. *elegans.*

The test substance can be a known chemical or pharmaceutical substance, such as, but not limited to, an anti-psychotic disorder therapeutic; or the test substance can be novel synthetic or natural chemical entity, or a combination of two or more of the aforesaid substances.

There is provided a method of identifying a substance capable of promoting or suppressing the generation of the analyte biomarker in a subject, comprising exposing a test cell to a test substance and monitoring the level of the analyte biomarker within said test cell, or secreted by said test cell.

The test cell could be prokaryotic, however a eukaryotic cell will suitably be employed in cell-based testing methods. Suitably, the eukaryotic cell is a yeast cell, insect cell, *Drosophila* cell, amphibian cell (e.g. from *Xenopus), C. elegans* cell or is a cell of human, non-human primate, equine, bovine, porcine, caprine, ovine, canine, feline, piscine, rodent or murine origin.

In methods for identifying substances of potential therapeutic use, non-human animals or cells can be used that are capable of expressing the analyte.

Screening methods also encompass a method of identifying a ligand capable of binding to the analyte biomarker, comprising incubating a test substance in the presence of the analyte biomarker in conditions appropriate for binding, and detecting and/or quantifying binding of the analyte to said test substance.

High-throughput screening technologies based on the biomarker, uses and methods of the invention, e.g. configured in an array format, are suitable to monitor biomarker signatures for the identification of potentially useful therapeutic compounds, e.g. ligands such as natural compounds, synthetic chemical compounds (e.g. from combinatorial libraries), peptides, monoclonal or polyclonal antibodies or fragments thereof, which may be capable of binding the biomarker.

Methods can be performed in array format, e.g. on a chip, or as a multiwell array. Methods can be adapted into platforms for single tests, or multiple identical or multiple non-identical tests, and can be performed in high throughput format. Methods of the invention may comprise performing one or more additional, different tests to confirm or exclude diagnosis, and/or to further characterise a condition.

Provided is a substance, e.g. a ligand, identified or identifiable by an identification or screening method. Such substances may be capable of inhibiting, directly or indirectly, the activity of the analyte biomarker, or of suppressing generation of the analyte biomarker. The term "substances" includes substances that do not directly bind the analyte biomarker and directly modulate a function, but instead indirectly modulate a function of the analyte biomarker. Ligands are also included in the term substances; ligands of the invention (e.g. a natural or synthetic chemical compound, peptide, aptamer, oligonucleotide, antibody or antibody fragment) are capable of binding, suitably specific binding, to the analyte.

Provided is a substance for use in the treatment of schizophrenia or other psychotic disorder, or predisposition thereto.

Also provided is the use of a substance in the treatment of schizophrenia or other psychotic disorder, or predisposition thereto.

Also provided is the use of a substance as a medicament.

Yet further provided is the use of a substance in the manufacture of a medicament for the treatment of schizophrenia or other psychotic disorder, or predisposition thereto.

A kit for diagnosing or monitoring schizophrenia or other psychotic disorder, or predisposition thereto is provided. Suitably a kit may contain one or more components selected from the group: a ligand specific for the analyte biomarker or a structural/shape mimic of the analyte biomarker, one or more controls, one or more reagents and one or more consumables; optionally together with instructions for use of the kit in accordance with any of the methods defined herein.

The identification of biomarkers for schizophrenia or other psychotic disorder permits integration of diagnostic procedures and therapeutic regimes. Currently there are significant delays in determining effective treatment and hitherto it has not been possible to perform rapid assessment of drug response. Traditionally, many anti-psychotic therapies have required treatment trials lasting weeks to months for a given therapeutic approach. Detection of an analyte biomarker can be used to screen subjects prior to their participation in clinical trials. The biomarkers provide the means to indicate therapeutic response, failure to respond, unfavourable side-effect profile, degree of medication compliance and achievement of adequate serum drug levels. The biomarkers may be used to provide warning of adverse drug response. Biomarkers are useful in development of personalized brain therapies, as assessment of response can be used to fine-tune dosage, minimise the number of prescribed medications, reduce the delay in attaining effective therapy and avoid adverse drug reactions. Thus by monitoring a biomarker, patient care can be tailored precisely to match the needs determined by the disorder and the pharmacogenomic profile of the patient, the biomarker can thus be used to titrate the optimal dose, predict a positive therapeutic response and identify those patients at high risk of severe side effects.

Biomarker-based tests provide a first line assessment of 'new' patients, and provide objective measures for accurate and rapid diagnosis, in a time frame and with precision, not achievable using the current subjective measures.

Furthermore, diagnostic biomarker tests are useful to identify family members or patients at high risk of developing schizophrenia or other psychotic disorder. This permits initiation of appropriate therapy, or preventive measures, e.g. managing risk factors. These approaches are recognised to improve outcome and may prevent overt onset of the disorder.

Biomarker monitoring methods, biosensors and kits are also vital as patient monitoring tools, to enable the physician to determine whether relapse is due to worsening of the disorder, poor patient compliance or substance abuse. If pharmacological treatment is assessed to be inadequate, then therapy can be reinstated or increased; a change in therapy can be given if appropriate. As the biomarkers are sensitive to the state of the disorder, they provide an indication of the impact of drug therapy or of substance abuse.

The following study illustrates the invention.

187 analytes were analysed in accordance with the protocol described in International Patent Application No. PCT/GB2008/004186 which included 6 separate cohorts, the details of which are as follows:
Cohorts 1, 2 and 3 are exactly as described in International Patent Application No. PCT/GB2008/004186 and are from the Universities of Cologne, Muenster and Magdeburg.
Cohort 4 is a population of patients and controls from the University of Magdeburg with full demographic details shown in Table 1:

**Table 1**

| **Demographic Details of Cohort 4** | | |
|---|---|---|
| **Cohort 4** | **Schizophrenia** | **Controls** |
| Number | 40 | 40 |
| Sex (Male/Female) | 27/13 | 26/14 |
| Age | 34.9 ± 9.9 | 35.5 ± 11.1 |

Cohort 5 is a population of patients and controls from the Erasmus University (Rotterdam) with full demographic details shown in Table 2:

**Table 2**

| **Demographic Details of Cohort 5** | | |
|---|---|---|
| **Cohort 5** | **Schizophrenia** | **Controls** |
| Number | 47 | 40 |
| Sex (Male/Female) | 36/11 | 33/7 |
| Age | 26.3 ± 7.5 | 26.8 ± 4.1 |

Cohort 6 is a cohort from the US military. It consists of bipolar disorder patients and controls with full demographic details shown in Table 3:

**Table 3**

| **Demographic Details of Cohort 6** | | |
|---|---|---|
| **Cohort 6** | **Bipolar Disorder** | **Controls** |
| Number | 110 | 110 |
| Sex (Male/Female) | 70/40 | 70/40 |
| Age | 21.3 ± 3.6 | 21.2 ± 3.5 |

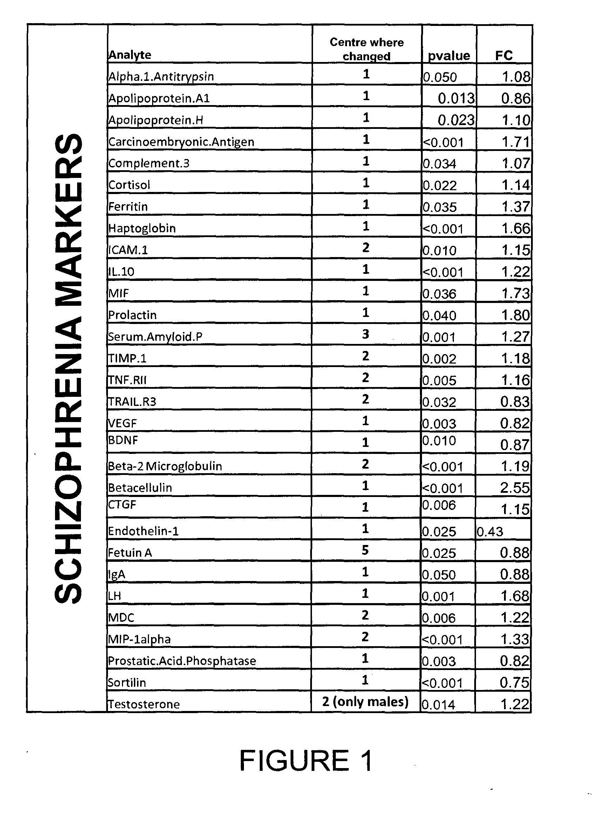

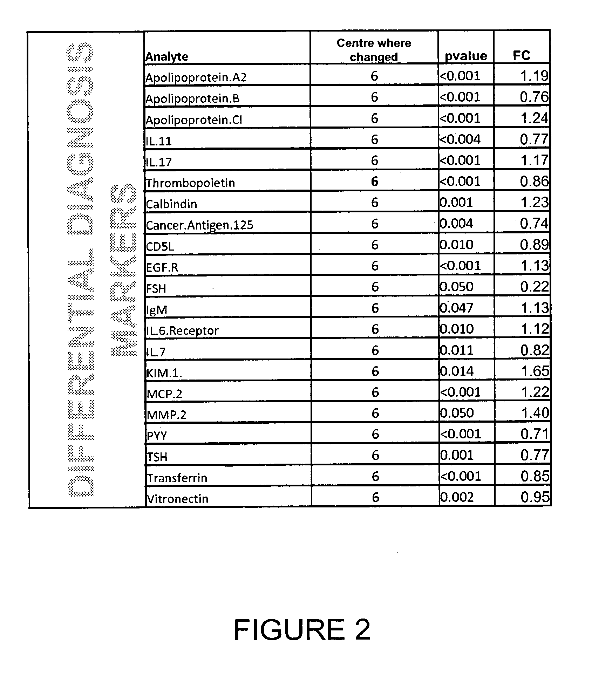

The results of this study identified 51 significant biomarkers, 30 of which were found to provide a sensitive diagnostic for schizophrenia (see Figure 1) and 21 which demonstrated effective diagnosis of bipolar disorder (see Figure 2).

Figure 3 describes the reproducibility of schizophrenia diagnosis across the 5 cohorts (i.e. Cohorts 1-3, 4 and 5) for the 30 schizophrenia markers listed in Figure 1, wherein the number of asterisks (*) indicate the number of cohorts in which the analyte is altered between schizophrenia patients and controls. The column marked "SZ" in Figure 3 also describes the observed effect of the analyte. For example, the black colour indicates increased levels of the analyte in the schizophrenia patients, the grey colour indicates decreased levels of the analyte in the schizophrenia patients and the white colour indicates no evidence for change.

Figure 4 describes analogous results to Figure 3 for the 21 differential diagnosis markers listed in Figure 2. All 21 markers featured statistically significant difference between bipolar disorder patients and matched controls from cohort 6.

## Claims

1. In vitro use of Alpha 1 Antitrypsin, Apolipoprotein A1, Cortisol, Ferritin and IgA as a specific panel of biomarkers for diagnosing or monitoring schizophrenia or predisposition thereto.

2. Use as defined in claim 1, wherein the panel additionally comprises Prolactin, Vitronectin, Sortilin, VEGF, IL-11, MIF, Endothelin-1, IL-7, Serum Amyloid P and IL-17.

3. Use as defined in claim 1, wherein the panel additionally comprises Brain Derived Neurotrophic Factor (BDNF), Haptoglobin, ICAM-1, MIF, Serum Amyloid P, Sortilin, Testosterone, Thyroid Stimulating Hormone (TSH), TNF RII, Transferrin and Vitronectin.

4. Use as defined in claim 3, wherein the panel additionally comprises Apolipoprotein A2, Beta-2 Microglobulin, EGF receptor, Alpha-2-HS-glycoprotein (Fetuin A), IgM, IL-6 receptor, MDC, TRAIL R3 and VEGF.

5. Use as defined in claim 3, wherein the panel additionally comprises Cancer antigen 125, Kidney injury molecule 1, MCP-2, Apolipoprotein H, Carcinoembryonic antigen (CEA), Complement 3, IL-10, Prolactin, Tissue Inhibitor of Metalloprotease-1 (TIMP 1), Betacellulin, Connective Tissue Growth Factor (CTGF), Endothelin-1, Luteinizing hormone (LH), MIP-1 alpha, Prostatic Acid Phosphatase, Apolipoprotein B, Apolipoprotein CI, IL-11, IL-17, Thrombopoeitin, Calbindin, CD5L, Follicle Stimulating Hormone (FSH), IL-7, MMP-2 and Peptide YY (PYY).

6. Use of Cancer antigen 125, Kidney injury molecule 1, MCP-2, Alpha 1 Antitrypsin, Apolipoprotein A1, Apolipoprotein H, Carcinoembryonic antigen (CEA), Complement 3, Cortisol, Ferritin, Haptoglobin, ICAM-1, IL-10, MIF, Prolactin, Serum Amyloid P, Tissue Inhibitor of Metalloprotease-1 (TIMP 1), TNF RII, TRAIL R3, VEGF, Brain Derived Neurotrophic Factor (BDNF), Beta-2 Microglobulin, Betacellulin, Connective Tissue Growth Factor (CTGF), Endothelin-1, Alpha-2-HS-glycoprotein (Fetuin A), IgA, Luteinizing hormone (LH), MDC, MIP-1 alpha, Prostatic Acid Phosphatase, Sortilin, Testosterone, Apolipoprotein A2, Apolipoprotein B, Apolipoprotein CI, IL-11, IL-17, Thrombopoeitin, Calbindin, CD5L, EGF receptor, Follicle Stimulating Hormone (FSH), IgM, IL-6 receptor, IL-7, MMP-2, Peptide YY (PYY), Thyroid Stimulating Hormone (TSH), Transferrin and Vitronectin as a specific panel of analyte biomarkers for differential diagnosis of schizophrenia over bipolar disorder.

7. A method of diagnosing or monitoring schizophrenia or predisposition thereto, comprising detecting and/or quantifying, in a sample from a test subject, the panel of biomarkers as defined in any of claims 1 to 5.

8. A method of monitoring efficacy of a therapy in a subject having, suspected of having, or of being predisposed to schizophrenia, comprising detecting and/or quantifying, in a sample from said subject, the panel of biomarkers as defined in any of claims 1 to 5.

9. A method as defined in claim 7 or claim 8, which is conducted on samples taken on two or more occasions from a test subject.

10. A method as defined in any of claims 7 to 9, comprising comparing the amount of the biomarkers present in said test sample with one or more controls.

11. A method as defined in any of claims 7 to 10, wherein quantifying is performed by measuring the concentration of the analyte biomarker in the or each sample.

12. A method as defined in any of claims 7 to 11, wherein detecting and/or quantifying is performed by one or more methods selected from SELDI (-TOF), MALDI (-TOF), a 1-D gel-based analysis, a 2-D gel-based analysis, Mass spec (MS), reverse phase (RP) LC, size permeation (gel filtration), ion exchange, affinity, HPLC, UPLC or other LC or LC-MS-based technique.

13. A method as defined in any of claims 7 to 12, wherein the biological sample is cerebrospinal fluid, whole blood, blood serum, plasma, urine, saliva, or other bodily fluid, or breath, condensed breath, or an extract or purification therefrom, or dilution thereof.

14. Use of a kit for monitoring or diagnosing schizophrenia, comprising a biosensor capable of detecting and/or quantifying the panel of biomarkers according to the use of claims 1 to 6.

## Patentansprüche

1. *In-vitro*-Verwendung von Alpha-1-Antitrypsin, Apolipoprotein A1, Cortisol, Ferritin und IgA als spezifische Gruppe von Biomarkern zum Diagnostizieren oder Überwachen von Schizophrenie oder einer Veranlagung dafür.

2. Verwendung nach Anspruch 1, wobei die Gruppe zusätzlich Prolaktin, Vitronektin, Sortilin, VEGF, IL-11, MIF, Endothelin-1, IL-7, Serum-Amyloid-P und IL-17 beinhaltet.

3. Verwendung nach Anspruch 1, wobei die Gruppe zusätzlich vom Gehirn stammenden neurotrophen Faktor (BDNF), Haptoglobin, ICAM-1, MIF, Serum-Amyloid-P, Sortilin, Testosteron, thyreoidastimulierendes Hormon (TSH), TNF RII, Transferrin und Vitronektin beinhaltet.

4. Verwendung nach Anspruch 3, wobei die Gruppe zusätzlich Apolipoprotein A2, Beta-2-Mikroglobulin, EGF-Rezeptor, Alpha-2-HS-Glykoprotein (Fetuin A), IgM, IL-6-Rezeptor, MDC, TRAIL R3 und VEGF beinhaltet.

5. Verwendung nach Anspruch 3, wobei die Gruppe zusätzlich Krebsantigen 125, Kidney Injury Molecule-1, MCP-2, Apolipoprotein H, carcinoembryonales Antigen (CEA), Komplement 3, IL-10, Prolaktin, Gewebeinhibitor von Metalloprotease-1 (TIMP-1), Betacellulin, Bindegewebewachstumsfaktor (CTGF), Endothelin-1, luteinisierendes Hormon (LH), MIP-1alpha, prostataspezifische saure Phosphatase, Apolipoprotein B, Apolipoprotein CI, IL-11, IL-17, Thrombopoeitin, Calbindin, CD5L, follikelstimulierendes Hormon (FSH), IL-7, MMP-2 und Peptid YY (PYY) beinhaltet.

6. Verwendung von Krebsantigen 125, Kidney Injury Molecule-1, MCP-2, Alpha-1-Antitrypsin, Apolipoprotein A1, Apolipoprotein H, carcinoembryonalem Antigen (CEA), Komplement 3, Cortisol, Ferritin, Haptoglobin, ICAM-1, IL-10, MIF, Prolaktin, Serum-Amyloid-P, Gewebeinhibitor von Metalloprotease-1 (TIMP-1), TNF RII, TRAIL R3, VEGF, vom Gehirn stammendem neurotrophem Faktor (BDNF), Beta-2-Mikroglobulin, Betacellulin, Bindegewebewachstumsfaktor (CTGF), Endothelin-1, Alpha-2-HS-Glykoprotein (Fetuin A), IgA, luteinisierendem Hormon (LH), MDC, MIP-1alpha, prostataspezifischer saurer Phosphatase, Sortilin, Testosteron, Apolipoprotein A2, Apolipoprotein B, Apolipoprotein CI, IL-11, IL-17, Thrombopoeitin, Calbindin, CD5L, EGF-Rezeptor, follikelstimulierendem Hormon (FSH), IgM, IL-6-Rezeptor, IL-7, MMP-2, Peptid YY (PYY), thyreoidastimulierendem Hormon (TSH), Transferrin und Vitronektin als spezifische Gruppe von Analyt-Biomarkern für eine Differentialdiagnose von Schizophrenie gegenüber bipolarer Störung.

7. Verfahren zum Diagnostizieren oder Überwachen von Schizophrenie oder einer Veranlagung dafür, das das Detektieren und/oder Quantifizieren der Gruppe von Biomarkern nach einem der Ansprüche 1 bis 5 in einer Probe von einem Testsubjekt beinhaltet.

8. Verfahren zum Überwachen der Wirksamkeit einer Therapie bei einem Subjekt, das Schizophrenie hat, vermutlich Schizophrenie hat oder vermutlich eine Veranlagung für Schizophrenie hat, das das Detektieren und/oder Quantifizieren der Gruppe von Biomarkern nach einem der Ansprüche 1 bis 5 in einer Probe von dem genannten Subjekt beinhaltet.

9. Verfahren nach Anspruch 7 oder Anspruch 8, das an Proben durchgeführt wird, die einem Testsubjekt bei zwei oder mehr Gelegenheiten entnommen werden.

10. Verfahren nach einem der Ansprüche 7 bis 9, das das Vergleichen der Menge der in der genannten Testprobe enthaltenen Biomarker mit einer oder mehreren Kontrollen beinhaltet.

11. Verfahren nach einem der Ansprüche 7 bis 10, wobei die Quantifizierung durch Messen der Konzentration des Analyt-Biomarkers in der oder jeder Probe erfolgt.

12. Verfahren nach einem der Ansprüche 7 bis 11, wobei das Detektieren und/oder Quantifizieren mit einem oder mehreren Verfahren erfolgt, die ausgewählt sind aus: SELDI (-TOF), MALDI (-TOF), einer 1D-gelbasierten Analyse, einer 2D-gelbasierten Analyse, Massenspektrometrie (MS), Umkehrphasen-(RP)-LC, Größenpermeation (Gelfiltration), Ionenaustausch, Affinität, HPLC, UPLC oder einer anderen LC- oder LC-MS-basierten Methode.

13. Verfahren nach einem der Ansprüche 7 bis 12, wobei die biologische Probe Rückenmarksflüssigkeit, Vollblut, Blutserum, Plasma, Urin, Speichel oder eine andere Körperflüssigkeit oder Atem, kondensierter Atem oder ein Extrakt oder Reinigungsprodukt davon oder ein Verdünnungsprodukt davon ist.

14. Verwendung eines Kits zum Überwachen oder Diagnostizieren von Schizophrenie, der einen Biosensor beinhaltet, der die Gruppe von Biomarkern gemäß der Verwendung in den Ansprüchen 1 bis 6 detektieren und/oder quantifizieren kann.

## Revendications

1. Utilisation *in vitro* de l'alpha-1-antitrypsine, de l'apolipoprotéine A1, du cortisol, de la ferritine et de l'IgA en tant que batterie spécifique de biomarqueurs pour le diagnostic ou le suivi de la schizophrénie ou d'une prédisposition à cette affection.

2. Utilisation selon la revendication 1, dans laquelle la batterie comprend également la prolactine, la vitronectine, la sortiline, le VEGF, l'IL-11, le MIF, l'endothéline-1, l'IL-7, l'amyloïde P sérique et l'IL-17.

3. Utilisation selon la revendication 1, dans laquelle la batterie comprend également le BDNF *(Brain Derived Neurotrophic Factor),* l'haptoglobine, l'ICAM-1, le MIF, l'amyloïde P sérique, la sortiline, la testostérone, la TSH (thyréostimuline), le récepteur TNF-RII, la transferrine et la vitronectine.

4. Utilisation selon la revendication 3, dans laquelle la batterie comprend également l'apolipoprotéine A2, la bêta-2-microglobuline, le récepteur à l'EGF, l'alpha-2-HS-glycoprotéine (fétuine A), l'IgM, le récepteur à l'IL-6, la MDC, le récepteur TRAIL-R3 et le VEGF.

5. Utilisation selon la revendication 3, dans laquelle la batterie comprend également l'antigène CA-125, la KIM-1 (molécule 1 associée à une lésion rénale), la MCP-2, l'apolipoprotéine H, l'antigène carcino-embryonnaire (ACE), le facteur C3 du complément, l'IL-10, la prolactine, le TIMP-1 (inhibiteur tissulaire de la métalloprotéinase-1), la bêta-celluline, le CTGF (facteur de croissance du tissu conjonctif), l'endothéline-1, la LH (gonadotrophine B), la MIP-1 alpha, la phosphatase acide prostatique, l'apolipoprotéine B, l'apolipoprotéine C-I, l'IL-11, l'IL-17, la thrombopoïétine, la calbindine, la CD5L, la FSH (gonadotrophine A), l'IL-7, la MMP-2 et le PYY (peptide YY).

6. Utilisation de l'antigène CA-125, de la KIM-1, de la MCP-2, de l'alpha-1-antitrypsine, de l'apolipoprotéine A1, de l'apolipoprotéine H, de l'antigène carcino-embryonnaire (ACE), du facteur C3 du complément, du cortisol, de la ferritine, de l'haptoglobine, de l'ICAM-1, de l'IL-10, du MIF, de la prolactine, de l'amyloïde P sérique, du TIMP-1 (inhibiteur tissulaire de la métalloprotéinase-1), du récepteur TNF-RII, du récepteur TRAIL-R3, du VEGF, du BDNF *(Brain Derived Neurotrophic Factor),* de la bêta-2-microglobuline, de la bêta-celluline, du CTGF (facteur de croissance du tissu conjonctif), de l'endothéline-1, de l'alpha-2-HS-glycoprotéine (fétuine A), de l'IgA, de la LH (gonadotrophine B), de la MDC, de la MIP-1 alpha, de la phosphatase acide prostatique, de la sortiline, de la testostérone, de l'apolipoprotéine A2, de l'apolipoprotéine B, de l'apolipoprotéine C-I, de l'IL-11, de l'IL-17, de la thrombopoïétine, de la calbindine, de la CD5L, du récepteur à l'EGF, de la FSH (gonadotrophine A), de l'IgM, du récepteur à l'IL-6, de l'IL-7, de la MMP-2, du PYY (peptide YY), de la thyréostimuline (TSH), de la transferrine et de la vitronectine en tant que batterie spécifique d'analytes biomarqueurs pour le diagnostic différentiel de schizophrénie par rapport à une maladie bipolaire.

7. Méthode de diagnostic ou de suivi de la schizophrénie ou d'une prédisposition à cette affection, qui comprend la détection et/ou quantification, dans un échantillon obtenu chez un sujet à tester, de la batterie de biomarqueurs selon l'une quelconque des revendications 1 à 5.

8. Méthode de suivi de l'efficacité d'un traitement chez un sujet atteint ou suspecté d'être atteint de schizophrénie ou d'une prédisposition à cette affection, qui comprend la détection et/ou quantification, dans un échantillon obtenu chez ledit sujet, de la batterie de biomarqueurs selon l'une quelconque des revendications 1 à 5.

9. Méthode selon la revendication 7 ou la revendication 8, qui est effectuée sur des échantillons prélevés à deux occasions ou plus chez un sujet à tester.

10. Méthode selon l'une quelconque des revendications 7 à 9, qui comprend la comparaison de la quantité de biomarqueurs présents dans ledit échantillon à tester et dans un ou plusieurs échantillons témoins.

11. Méthode selon l'une quelconque des revendications 7 à 10, dans laquelle la quantification est effectuée en mesurant la concentration de l'analyte biomarqueur dans l'échantillon ou dans chaque échantillon.

12. Méthode selon l'une quelconque des revendications 7 à 11, dans laquelle la détection et/ou la quantification est effectuée en utilisant une ou plusieurs méthodes sélectionnées parmi les suivantes : SELDI (-TOF), MALDI (-TOF), analyse sur gel 1-D, analyse sur gel 2-D, spectrométrie de masse (SM), chromatographie en phase inverse (PI), chromatographie par perméation de gel (filtration sur gel), chromatographie par échange d'ions, chromatographie d'affinité, CLHP, UPLC ou autre technique reposant sur la CL ou la CL-SM.

13. Méthode selon l'une quelconque des revendications 7 à 12, dans laquelle l'échantillon biologique est le liquide céphalorachidien, le sang entier, le sérum sanguin, le plasma, l'urine, la salive ou tout autre liquide corporel ou l'haleine, l'haleine condensée ou un extrait, une forme purifiée ou une forme diluée de ceux-ci.

14. Utilisation d'un kit pour le suivi ou le diagnostic de la schizophrénie, qui comprend un biocapteur capable de détecter et/ou quantifier la batterie de biomarqueurs selon l'utilisation des revendications 1 à 6.
